# EUROPÄISCHE PATENTSCHRIFT

(11) **EP 1 134 584 B1**
(45) Veröffentlichungstag und Bekanntmachung des Hinweises auf die Patenterteilung: **20.07.2005**
(21) Anmeldenummer: 01102952.7
(22) Anmeldetag: 08.02.2001
(51) Int. Cl.: G01N 33/50

(54) **Induzierte Aggregation und Agglutination von Plättchen**
Induced Aggregation and Agglutination of Platelets
Agglomération et Agglutination de Plaquettes induite

(30) Priorität: 17.03.2000 DE 10013377
(43) Veröffentlichungstag der Anmeldung: 19.09.2001
(73) Patentinhaber: Dade Behring Marburg GmbH, 35001 Marburg (DE)
(72) Erfinder: Patzke, Jürgen, Dr., 35039 Marburg (DE)

(56) Entgegenhaltungen:
- DE-A- 3 334 170
- US-A- 4 788 139
- US-A- 5 246 832
- US-A- 5 569 590
- US-A- 5 637 452
- US-A- 5 854 005
- YOKO MINAMOTO, TAKAAKI HATO, SHINGO NAKATANI, SHIGERU FUJITA: "Detection of Platelet Adhesion/aggregation to Immobilized Ligands on Microbeads by an Aggregometer" THROMBOSIS AND HAEMOSTASIS, Bd. 76, Nr. 6, - 1996 Seiten 1072-9, XP002921232 Stuttgart

## Beschreibung

Gegenstand der Erfindung ist ein Verfahren zur Messung der Aggregation oder Agglutination von Plättchen, wobei ein Reaktionsgemisch in einer ersten Reaktionsphase durchmischt wird und in einer auf die erste folgenden, zweiten Reaktionsphase weniger intensiv oder gar nicht durchmischt wird und die Messung bevorzugterweise in der zweiten Reaktionsphase erfolgt.

Blut besteht im wesentlichen aus dem Blutplasma, einer Lösung von niedermolekularen Substanzen und Proteinen, und zellulären Bestandteilen, von denen die Erythrozyten (rote Blutkörperchen), Leukozyten (weiße Blutkörperchen) und Thrombozyten (Blutplättchen) den größten Anteil ausmachen.

Thrombozyten haben in vivo hauptsächlich die Funktion, auf eine Verletzung schnell zu reagieren und die ersten Schritte zum Wundverschluß einzuleiten. Sie werden durch verschiedene Faktoren aktiviert und im Zuge der Aktivierung werden sie "klebrig", wie man es früher treffend nannte. Sie adherieren dann sowohl an verletzte Oberflächen als auch aneinander. Die Adhäsion aneinander nennt man Aggregation, wenn sie aktiv stattfindet, und Agglutination, wenn sie passiv, das heißt ohne physiologische Aktivität, abläuft.

Im Folgenden und insbesondere auch bei den Ansprüchen ist bei Benutzung des Terminus "Aggregation" sowohl die Aggregation als auch die Agglutination von Plättchen gemeint.

Die Fähigkeit zur Aggregation ist also wichtig für den Wundverschluß. Sie kann durch eine Vielzahl von Faktoren verstärkt oder verringert sein. Dazu gehören z.B. Krankheiten, Operationen, Medikamenteneinnahmen und die Zahl der Thrombozyten. Eine Verstärkung der Aggregationsneigung kann zu Thrombosen und eine Verringerung der Aggregationsneigung kann zu Blutungen führen.

Zur Untersuchung der Aggregationseigenschaften von Plättchen wurde von O'Brian (J. Clin. Path., 1962, 15, 452-455) und von Born (J. Physiol., 1963, 168, 178-195) eine Methode zur Plättchenaggregationsmessung entwickelt, die sich in den folgenden Jahrzehnten in der klinischen Diagnostik durchgesetzt hat (Bick RL, Clinics in Laboratory Medicine, Thrombosis and Hemostatis for the clinical laboratory: Part II, Volume 15, 1, 1-38). Bei der Methode wird zuerst Plättchen-reiches Plasma hergestellt. Im Ansatz wird zumeist ein Aktivator dazugegeben und unter permanentem Rühren die Extinktion verfolgt. Die Bildung der Aggregate kann durch die Abnahme der Extinktion quantitativ verfolgt werden. Zur Messung werden Spezialgeräte, sogenannte Aggregometer, verwendet, da übliche Photometer nicht über einen kontrollierten Rührmechanismus und angepaßte Auswertemodi verfügen.

Der Vorteil der Methode besteht in der quantitativen und kinetischen Messung der Aggregation und in der relativ leichten Automatisierbarkeit. Von Nachteil ist es, daß spezialisierte Geräte mit einem Rührmechanismus während der Messung benötigt werden. Diese Geräte, die Aggregometer, sind teuer und die Anschaffung lohnt sich nicht für jedes Labor. Da die Anzahl der Messungen oft limitiert ist, haben sich auch keine vollautomatisierten Geräte durchgesetzt und die Abarbeitung ist entsprechend umständlich. Auf der anderen Seite existieren eine Vielzahl von Analyseautomaten mit sehr hohem Automatisierungsgrad, die auf Grund der photometrischen Meßeinheit prinzipiell die Extinktionsabnahme messen könnten, aber nicht über die notwendige permanente Rührmöglichkeit verfügen.

Für die Messung der Plättchenaggregation ist nach derzeitigem Stand der Technik ein permanentes Rühren notwendig. Wie bereits die Arbeit von Born (1963) zeigte, ist die Aggregationsgeschwindigkeit stark von der Rührgeschwindigkeit abhängig. Aktuellere, methodische Übersichtsartikel setzten die Notwendigkeit des Rührens als selbstverständlich voraus (Yardumian DA, J Clin Pathol, 39, 1986, 701 - 712.

Bick RL, Thrombosis and Hemostasis for the Clinical Laboratory: Part II, 15, 1995, 1 - 38).

Das Rühren bewirkt vermutlich durch eine Erhöhung der Kollisionsfrequenz die Bildung größerer Aggregate, sogenannter Makroaggregate. Makroaggregate sind Aggregate von Plättchen, die zu einem Extinktionsabfall im Aggregometer führen. Sie sind deutlich größer als Mikroaggregate, deren Bildung entweder durch keine Extinktionsänderung oder sogar durch einen Extinktionsanstieg angezeigt wird (Kitek A. und Bredding K., 1980, Thromb & Haemost, 44, 1980, 3, 154-158). Mikroaggregate, bestehend aus 2-3 Plättchen, entstehen auch ohne Rühren (Longmire K. and Frojmovic M., Biophys J, 1990, 58(2):299-307). Diese Mikroaggregate sind aber in herkömmlichen Aggregometern nicht sichtbar (Toghi et al., Thromb Haemost, 1996, 75(5):38-43).

Auch die Bildung der Makroaggregate im von Willebrand Ristocetin-Kofaktor-Test läuft sehr ähnlich ab, wie bei der Aggregation von Plättchen in frischem Plättchen-reichem Plasma. Bei dem Ristocetin-Kofaktor-Test werden fixierte Thrombozyten durch die Zugabe von Plasma, das das von Willebrand-Protein enthält, und Ristocetin zur Agglutination gebracht (Weiss HJ et al., J Clin Invest, 1973, 52, 2708-2716.). Auch im Ristocetin-Kofaktor-Test zeigt die turbidimetrische Messung unter Rühren hauptsächlich die Bildung der Makroaggregate an (Kitek A. und Breddin K., 1980, Thromb & Haemost, 44, 1980, 3, 154-158. Toghi et al., Thromb Haemost, 1996, 75(5):38-43).

Der vorliegenden Erfindung lag also die Aufgabe zugrunde, ein Messverfahren zu finden, mittels dessen eine Plättchenaggregation auch ohne permanentes Rühren gemessen werden kann.

Gelöst wird diese Aufgabe durch das erfindungsgemäße Verfahren zur Messung der Aggregation von Blutplättchen (Plättchen), wobei ein Reaktionsgemisch aus Plättchen, bevorzugt physiologisch aktive Plättchen oder fixierte Plättchen, mit anderen Testansatzkomponenten in einer ersten Reaktionsphase durchmischt wird und in einer auf die erste folgenden, zweiten Reaktionsphase weniger intensiv oder gar nicht durchmischt wird. Die Messung der Plättchenaggregation oder -agglutination erfolgt bevorzugt in der zweiten Reaktionsphase. Weitere bevorzugte Ausführungsformen der Erfindung sind in den Ansprüchen 2-17 näher beschriebenen.

Überraschenderweise wurde gefunden, daß die Bildung von Makroaggregaten nicht unbedingt des permanenten Rührens bedarf, sondern daß ein kurze Rührzeit nach Zugabe des Plättchenaktivators ausreicht, um eine Bildung von Makroaggregaten zu induzieren. Die Bildung der Makroaggregate erfolgt nach der Induktionsphase allein unter Einwirkung der Brownschen Diffusion.

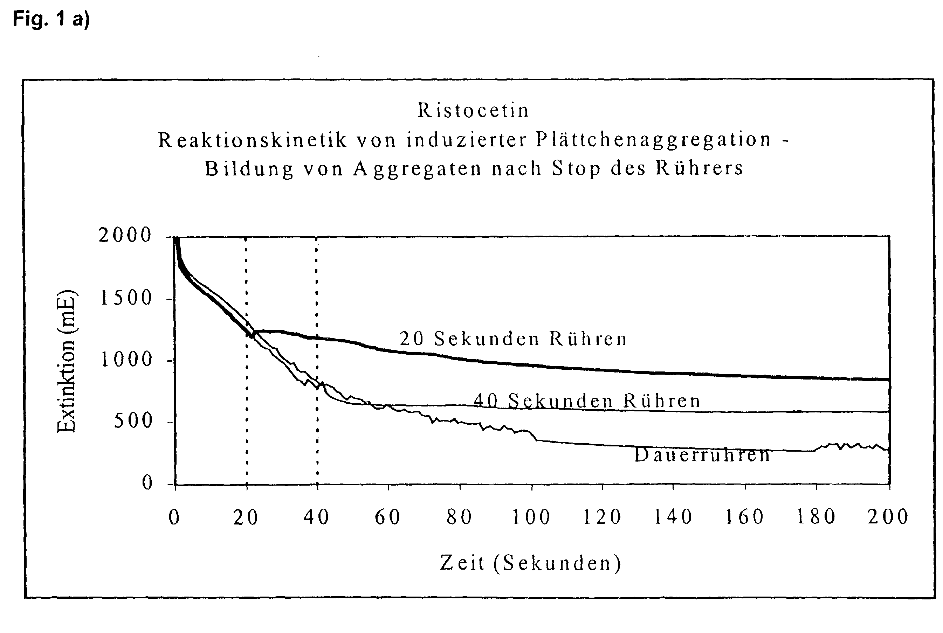

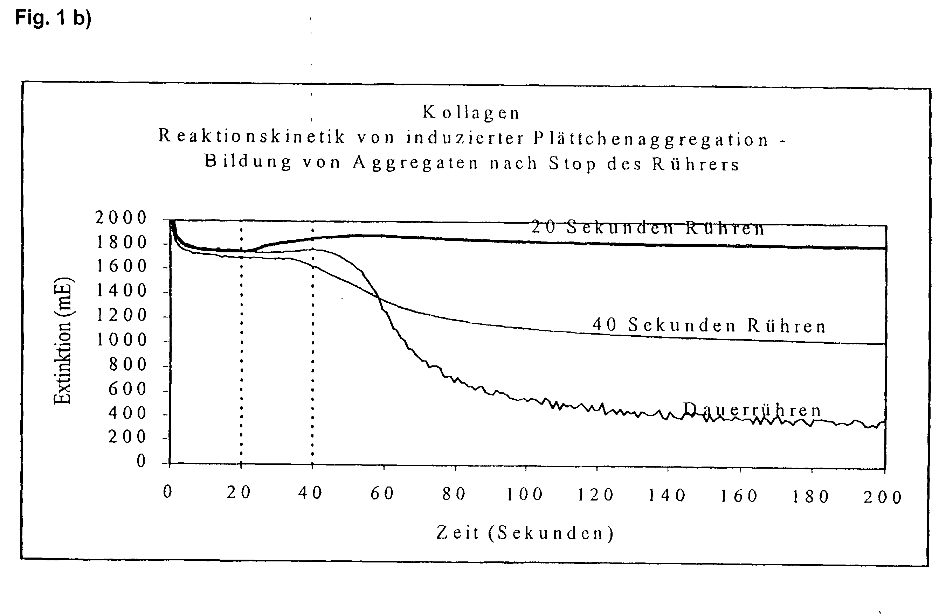

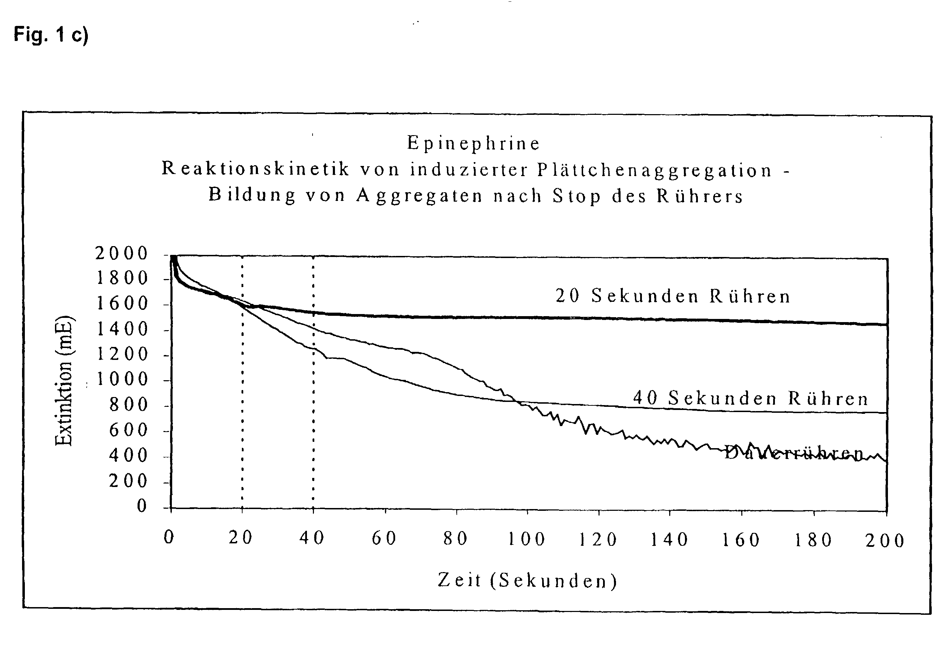

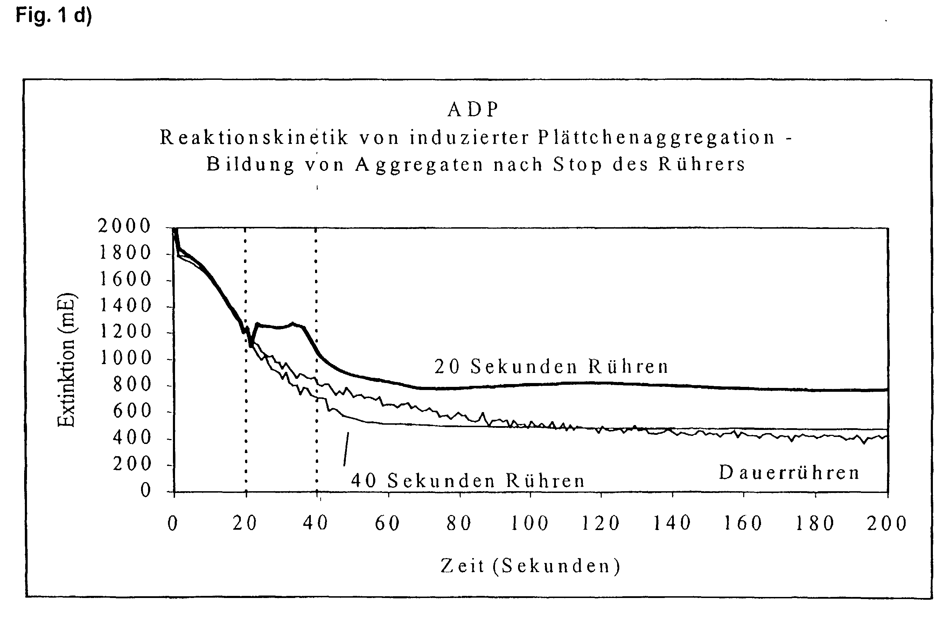

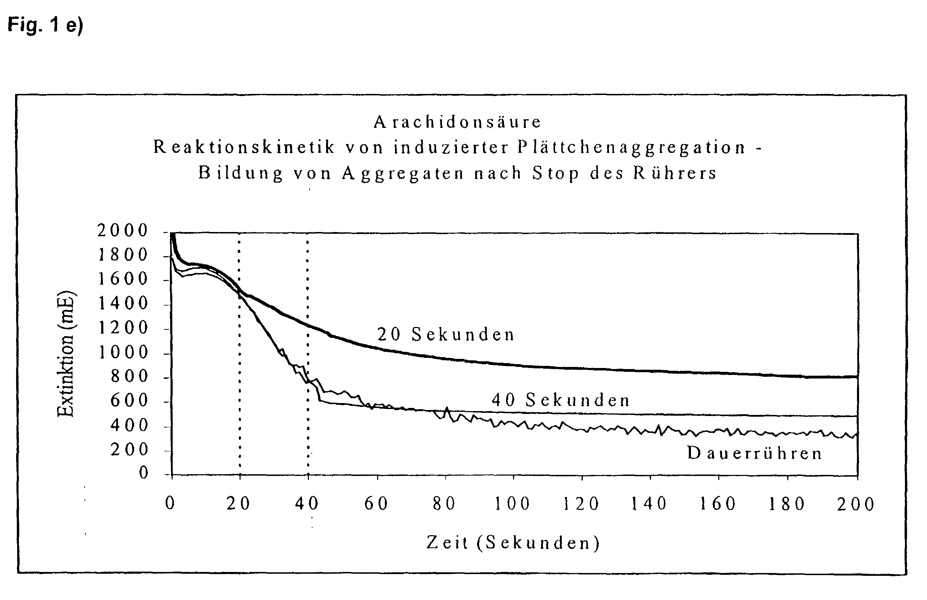

Die Induktion der Makroaggregatbildung ist bei Benutzung verschiedener Aktivatoren möglich (Abb. 1). Solche Aktivatoren wie z.B. Ristocetin, Kollagen, ADP oder Epinephrin sind dem Fachmann auf dem Gebiet der Thrombozytenaggregation hinlänglich bekannt. Eine Zusammfassung der Funktionsweise verschiedener Aktivatoren und ihrer Rezeptoren findet sich z.B. in Blockmans D. et al., Blood Review, 9, 1995, 143-1556. Die Reaktionskinetik der Bildung der Makroaggregate durch Brownsche Diffusion ist bei den verschiedenen Aktivatoren sehr unterschiedlich. Für die Bildung der Makroaggregate ist einerseits die Frequenz von Kollisionen von Mikroaggregaten mit anderen Mikroaggregaten oder einzelnen Plättchen entscheidend. Andererseits ist es wichtig, daß eine Kollision "erfolgreich" ist, wobei wiederum der Aktivierungszustand, die Ladungsverhältnisse, die Größe und Zahl der Pseudopodien und viele weitere Parameter eine Rolle spielen. Da die Aktivatoren, im folgenden auch Agonisten genannt, einen sehr unterschiedlichen Ablauf der Aktivierung bewirken, ist es verständlich, dass gerade die Makroaggregatbildung nach Stopp des Rührers bei den verschiedenen Agonisten sehr unterschiedlich ausfällt. Der Aktivierungszustand in dem Augenblick, in dem das Rühren gestoppt wird, ist sicherlich ein wichtiger Parameter für die Stärke der daraufhin ablaufenden Reaktion.

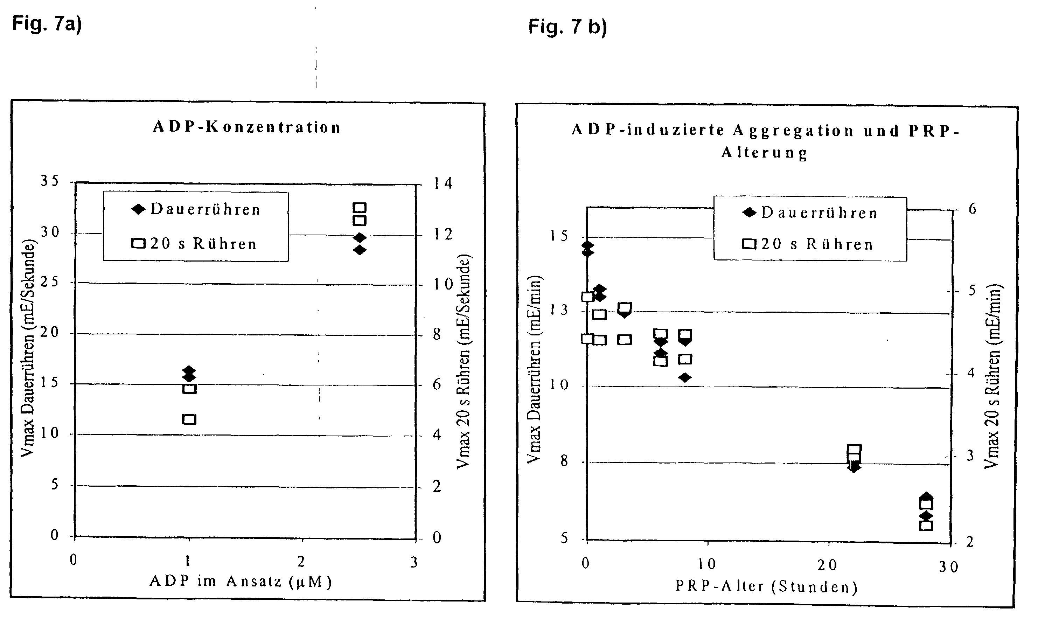

Die klassische Aggregationsmessung (Dauerrühren) und die induzierten Aggregationsmessung (Rühren nur zu Beginn) eignen sich beide, um die Aktivität von Thrombozyten zu erfassen. Beispielhaft wurden dazu sowohl die Konzentrationsabhängigkeit vom Agonisten als auch die Alterung von Plättchen-reichem Plasma untersucht. Die Aggregationsgeschwindigkeit ohne Dauerrühren liegt auf einem niedrigerem Niveau. Sie reagiert aber auf eine Konzentrationsänderung des Agonisten ADP und auf ein Alterung von Plättchen-reichem Plasma (bei Raumtemperatur) auf sehr ähnliche Weise (Abb. 7).

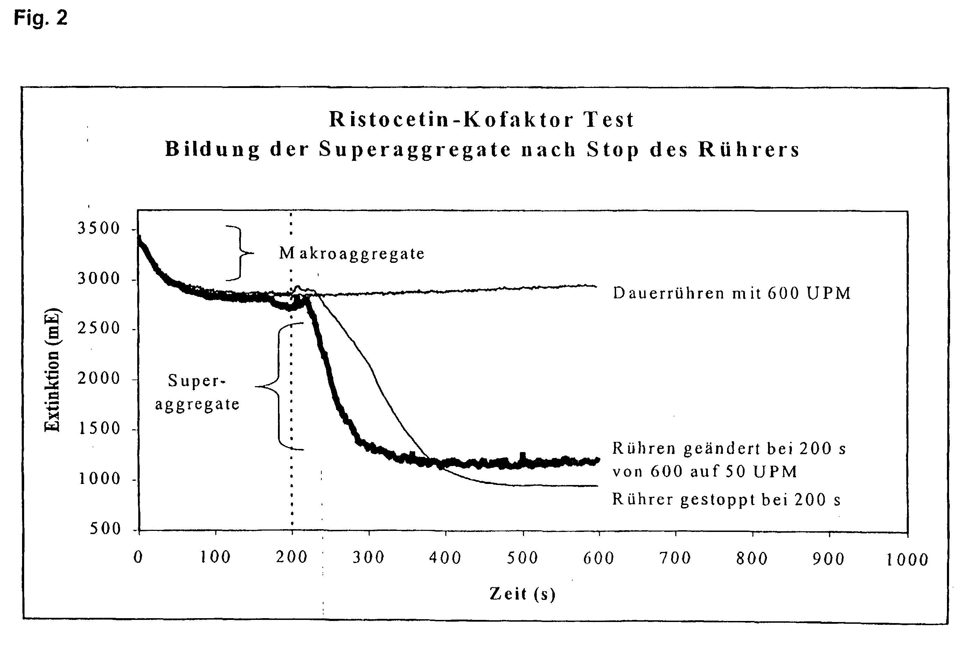

Überraschenderweise wurde auch gefunden, daß das Rühren nicht nur die Bildung von Makroaggregaten ermöglicht, sondern auch für die gegenteilige Reaktion, die Zerstörung von Makroaggregaten, verantwortlich sein kann. Das scheinbare Ende einer Aggregationsreaktion, typischerweise nach 5-10 Minuten, kann in Wirklichkeit ein Gleichgewichtszustand zwischen Bildung und Zerstörung von Makroaggregaten sein. Dies wird deutlich, wenn man in einem typischen vWF:RCo (von Willebrand Ristocetin-Kofaktor)-Test nach Abschluß eines Großteils der Reaktion das Rühren aussetzt. Kurze Zeit später setzt ein starker Extinktionsabfall ein, was auf die Bildung von besonders großen Makroaggregaten, die im folgenden Superaggregate genannt werden sollen, hindeutet (Abb. 2). Der Extinktionsabfall ist in etwa gleich groß wie bei einem sehr schwachen Rühren mit 50 UPM (Umdrehungen pro Minute), wodurch klar wird, daß nicht etwa die Sedimentation von Aggregaten den Extinktionsabfall bewirkt. Da beim vWF:RCo-Test die Thrombozyten Formalin-fixiert sind, ist auch ausgeschlossen, daß eine mit Verzögerung einsetzende biochemische Reaktion die Aggregatbildung bewirkt.

Unter Rühren im Zusammenhang mit der vorliegenden Erfindung sind auch die anderen, dem Fachmann bekannten Methoden der Durchmischung von Flüssigkeiten zu verstehen, wie zum Beispiel die Durchmischung durch Rühren, Schütteln, Ultraschall oder Vibration. Die Durchmischung kann auch durch die Bewegung in einem Zentrifugalanalysator oder durch Umpumpen des Reaktionsgemisches durch einen Pipettor oder die forcierte Zugabe der Reagenzien in das Reaktionsgefäß erfolgen.

Die im erfindungsgemäßen Verfahren bevorzugte Rührgeschwindigkeit liegt zwischen 200 und 2000 UPM, besonders bevorzugt zwischen 400 und 1200 UPM und ganz besonders bevorzugt zwischen 400 und 800 UPM.

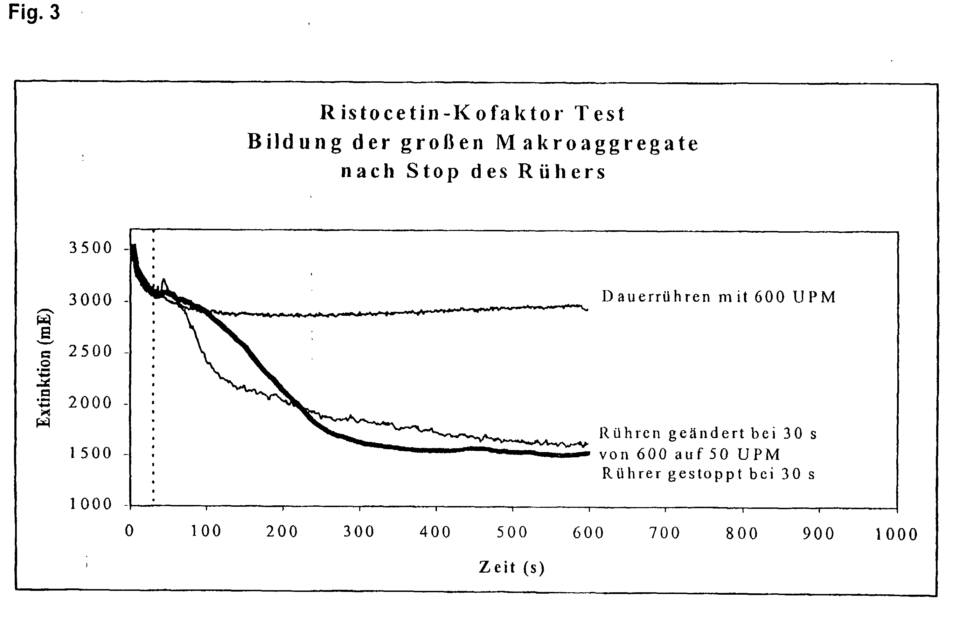

Der Bildung der Superaggregate wird bereits nach einer relativ kurzen Zeit (z.B. 30 Sekunden) induziert (Abb. 3).

Die Superaggregatbildung, ermöglicht durch reine Diffusion, ist wie die Aggregatbildung, die durch das Rühren herbeigeführt wird, in Geschwindigkeit und Endpunkt abhängig von der Von Willebrand-Konzentration (Abb. 4, Standard Human Plasma Verdünnungen). Dadurch kann eine Aggregationsmessung methodisch auch so durchgeführt werden, daß das Reaktionsgemisch kurze Zeit gerührt (oder auf andere Weise durchmischt) wird und dann erst die Messung der Extinktion erfolgt. Bereits sehr kurze Rührzeiten von wenigen Sekunden können ein Superaggregatbildung induzieren (Abb. 5). Auch bei diesen kurzen Zeiten ist ein Konzentrationsabhängigkeit im Ristocetin-Kofaktor-Test gegeben und die Präzision der Messungen ist mit denen bei langen Rührzeiten vergleichbar (Abb. 6).

Damit ergibt sich die Möglichkeit, den Ristocetin-Kofaktor-Test und die Plättchenaggregation auf allen Analyseautomaten zu adaptieren, bei denen eine genügende und reproduzierbare Durchmischung des Ansatzes erfolgen kann und danach eine Messung der Aggregatbildung, zum Beispiel durch Turbidimetrie oder Nephelometrie, durchgeführt werden kann. Die Auswertung kann sowohl kinetisch oder über andere (z.B. Start und/oder Endpunkt-) Methoden oder auch kombinierte Methoden erfolgen.

Man kann auch die Stabilität von Plättchenaggregaten durch den Vergleich der Aggregation in dem erfindungsgemäßen Verfahren mit einer analogen Messung bei ständigem Rühren feststellen. Bei instabilen Aggregaten ist die Bildung von Aggregaten ohne dauerhaftes Rühren relativ stark im Verhältnis zur Bildung von Aggregaten bei Dauerrühren. Ebenso kann das Verhältnis der Bildung von Aggregaten ganz ohne Rühren zur Bildung von Aggregaten bei kurzzeitigem oder dauerhaften Rühren von Bedeutung sein. Dadurch können wichtige zusätzliche diagnostische Informationen darüber gewonnen werden, wie wichtig unter verschiedenen physiologischen Bedingungen das Ausmaß von Strömungen auf die Aktivierung und Aggregatbildung sowie auf die Aggregatstabilität von Thrombozyten ist.

Im Ristocetin-Kofaktor-Test kann dadurch die Fähigkeit des von Willebrand Faktors oder anderer Plasmakomponenten, stabile Aggregate zu bilden untersucht werden.

Darüberhinaus kann durch sich abwechselnde Phasen von Rühren und Nicht-Rühren die Entstehung und die Auflösung von Aggregaten gemessen werden. Damit können wichtige Informationen über die Dynamik solcher Reaktionen gesammelt werden.

Die Dauer der aktiven Durchmischung hängt wesentlich von der Art des Assays ab, sie sollte zum Beispiel bei der Bestimmung der Plättchenaggregation 10-60 Sekunden betragen, beim Ristocetin-Kofaktor-Test 5-60 Sekunden, allgemein kann man sagen, daß sie nicht länger als 50 %, bevorzugterweise zwischen 1 und 40 %, ganz besonders bevorzugterweise zwischen 2 und 25 % der gesamten Meßzeit dauern kann.

Die Aggregatbildung beim Ristocetin-Kofaktor-Test findet mit physiologisch inaktiven Plättchen statt. Somit können auch andere Partikel, an deren Oberfläche Rezeptormoleküle die Aggregatbildung mit Plättchen oder mit anderen Partikeln direkt oder indirekt über Liganden vermitteln, im erfindungsgemäßen Verfahren zu Anwendung kommen.

Die folgenden Beispiele sollen lediglich die Erfindung erläutern, sie aber in keiner Weise einschränken.

### Beispiel 1)

### Bestimmung der Plättchenaggregation mit Ristocetin als Agonisten (Abb. 1a)

135 µl Plättchenreiches Plasma wurden mit 15 µl Ristocetin-Lösung (15 mg/ml) gemischt und 20 bzw. 40 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt (Schreiner, W. et al. Comput. Biol. Med., Vol. 21, No. 6, 435-441, 1991).

### Beispiel 2)

### Bestimmung der Plättchenaggregation mit Kollagen als Agonisten (Abb. 1b)

135 µl Plättchenreiches Plasma wurden mit 15 µl Kollagen-Lösung (2000 µg/ml) gemischt und 20 bzw. 40 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 3)

### Bestimmung der Plättchenaggregation mit Epinephrin als Agonisten (Abb. 1c)

135 µl Plättchenreiches Plasma wurden mit 15 µl Epinephrin-Lösung (100 µM) gemischt und 20 bzw. 40 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 4)

### Bestimmung der Plättchenaggregation mit ADP als Agonisten (Abb. 1 d)

135 µl Plättchen-reiches Plasma wurden mit 15 µl ADP-Lösung (200 µM) gemischt und 20 bzw. 40 Sekunden bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 5)

### Bestimmung der Plättchenaggregation mit Arachidonsäure als Agonisten (Abb. 1 e)

135 µl Plättchen-reiches Plasma wurden mit 15 µl Arachidonsäure-Lösung (5000 µg/ml) gemischt und 20 bzw. 40 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 6)

### Bestimmung der von Willebrand-Ristocetin-Kofaktoraktivität nach 200 Sek. Rührzeit (Abb. 2)

20 µl Plasma wurden mit 20 µl Imidazol-Puffer und 150 µl von Willebrand Reagenz (Dade Behring, Marburg, ca. 1.2 Mio formalin-fixierte Thrombozyten/µl, 1.2 mg/ml Ristocetin) gemischt und 200 Sekunden bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 7)

### Bestimmung der von Willebrand-Ristocetin-Kofaktoraktivität nach 30 Sek. Rührzeit (Abb. 3)

20 µl Plasma wurden mit 20 µl Imidazol-Puffer und 150 µl von Willebrand Reagenz (Dade Behring, Marburg, ca. 1.2 Mio formalin-fixierte Thrombozyten/µl, 1.2 mg/ml Ristocetin) gemischt und 30 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 8)

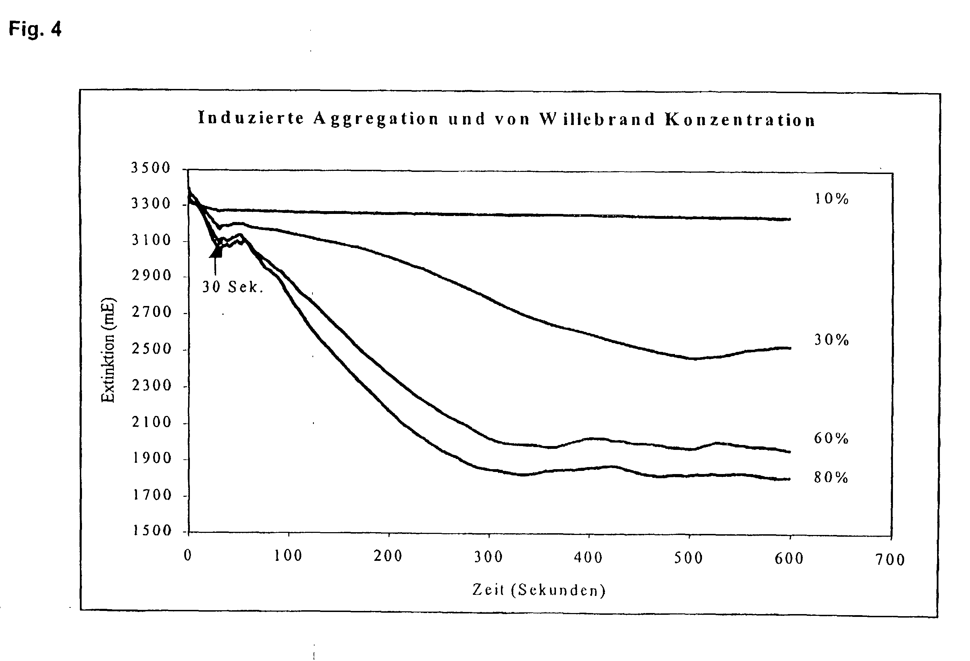

### Bestimmung der von Willebrand-Ristocetin-Kofaktoraktivität von Standardhumanplasma-Verdünnungen nach 30 Sek. Rührzeit (Abb. 4)

20 µl Plasma wurden mit Imidazol-Puffer in verschiedenen Stufen verdünnt (100% = unverdünnt), mit weiteren 20 µl Imidazol-Puffer und 150 µl von Willebrand Reagenz (Dade Behring, Marburg, ca. 1.2 Mio formalin-fixierte Thrombozyten/µl, 1.2 mg/ml Ristocetin) gemischt und 30 Sekunden bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 9)

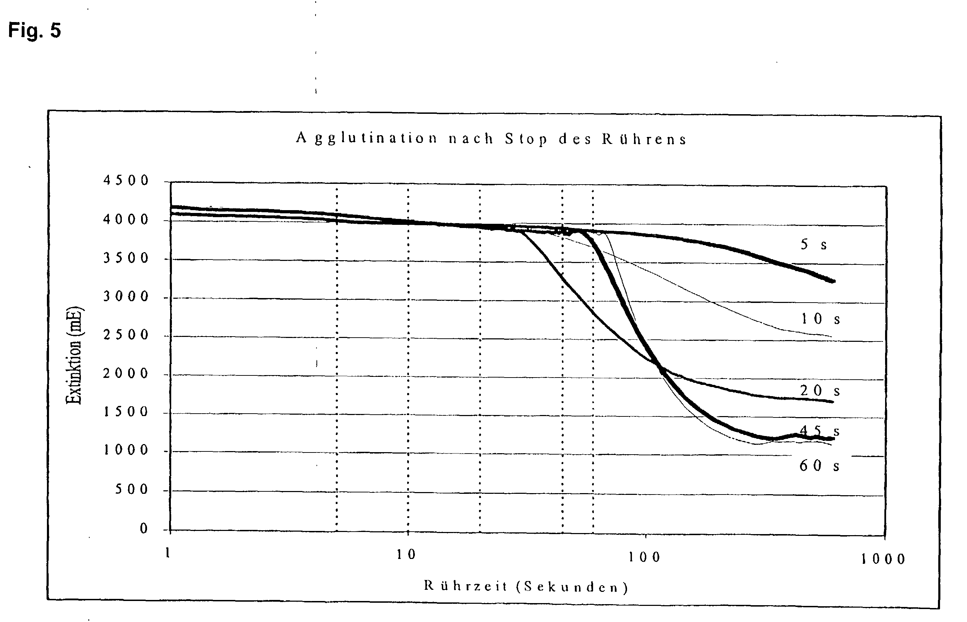

### Bestimmung der von Willebrand-Ristocetin-Kofaktoraktivität nach 5-60 Sek. Rührzeit (Abb. 5)

40 µl Plasma wurden mit 100 µl von Willebrand Reagenz (Dade Behring, Marburg, ca. 2.4 Mio formalin-fixierte /µl, 2.4 mg/ml Ristocetin) gemischt und 5, 10, 20, 45 oder 60 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 10)

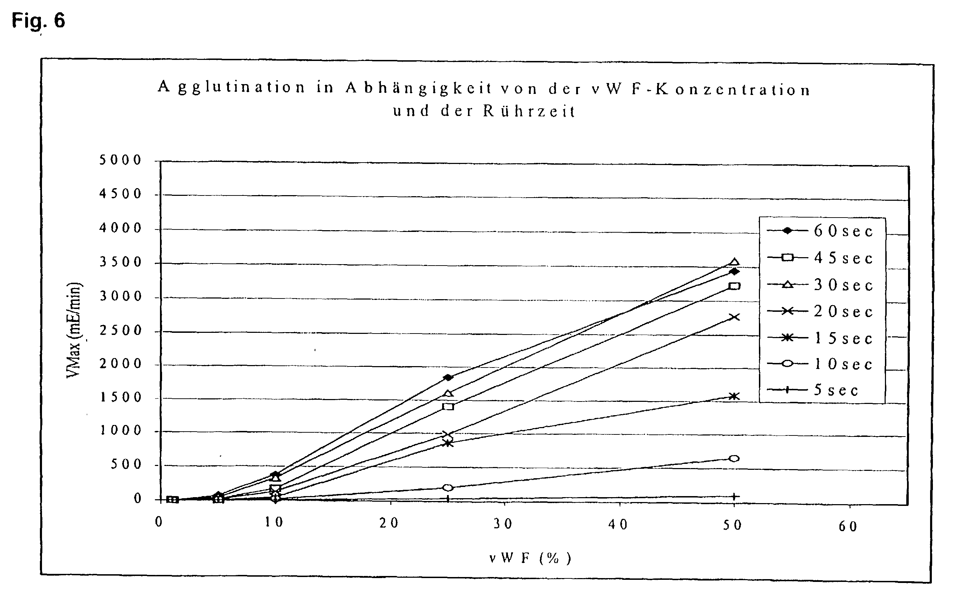

### Ermittlung von Kalibrationskurven mit Standard-Human-Plasma-Verdünnungen nach 5-60 Sek. Rührzeit (Abb. 6)

40 µl Standard-Human-Plasma (ca. 100% vWF) wurde mit Imidazol-Puffer verdünnt (Stufen 50, 25, 10, 5, 1 %) und mit 100 µl von Willebrand Reagenz (Dade Behring, Marburg, ca. 2.4 Mio formalin-fixierte Thrombozyten/µl, 2.4 mg/ml Ristocetin) gemischt und 5, 10, 20, 45 oder 60 Sek. bei 600 UPM gerührt. Die Auswertung erfolgte nach dem Stopp des Rührers. Zur Auswertung wurde die maximale Aggregationsgeschwindigkeit (Vmax der Extinktionsabnahme) nach dem Stopp des Rührers bestimmt.

### Beispiel 11)

### Bestimmung der ADP-Konzentrationsabhängigkeit der Plättchenaggregation mit und ohne Dauerrühren (Abb. 7, linkes Diagramm)

135 µl Plättchenreiches Plasma wurden mit 15 µl ADP-Lösung (10 bzw. 25 µM) gemischt und dauerhaft bzw. 20 Sekunden bei 600 UPM gerührt. Die Auswertung erfolgte direkt nach dem Mischen (Dauerrühren) bzw. nach dem Stopp des Rührers bei 20 Sekunden. Zur Auswertung wurde die maximale Aggregationsgeschwindigkeit (Vmax der Extinktionsabnahme) bestimmt.

### Beispiel 12)

### Bestimmung des Verlusts an Aktivität durch Lagerung von Plättchen-reichem Plasma bei Raumtemperatur durch Messung der Aggregation mit ADP als Agonist mit und ohne Dauerrühren (Abb. 7, rechtes Diagramm)

135 µl Plättchenreiches Plasma wurden mit 15 µl ADP-Lösung (200 µM) gemischt und dauerhaft bzw. 20 Sekunden bei 600 UPM gerührt. Die Auswertung erfolgte direkt nach dem Mischen (Dauerrühren) bzw. nach dem Stopp des Rührers bei 20 Sekunden. Zur Auswertung wurde die maximale Aggregationsgeschwindigkeit (Vmax der Extinktionsabnahme) bestimmt.

### Abbildungen:

**Fig. 1 a-e)**
   Bestimmung der induzierten Plättchenaggregation mit unterschiedlichen Agonisten
**Fig. 2)**
   Bestimmung der von Willebrand-Ristocetin-Kofaktoraktivität nach 200 Sek. Rührzeit
**Fig. 3)**
   Bestimmung der von Willebrand-Ristocetin-Kofaktoraktivität nach 30 Sek. Rührzeit
**Fig. 4)**
   Bestimmung der von Willebrand-Ristocetin-Kofaktoraktivität von Standardhumanplasma-Verdünnungen nach 30 Sek. Rührzeit
**Fig. 5)**
   Bestimmung der von Willebrand-Ristocetin-Kofaktoraktivität nach 5-60 Sek. Rührzeit
**Fig. 6)**
   Ermittlung von Kalibrationskurven mit Standard-Human-Plasma-Verdünnungen nach 5-60 Sek. Rührzeit
**Fig. 7a)**
   Bestimmung der ADP-Konzentrationsabhängigkeit der Plättchenaggregation mit und ohne Dauerrühren
**Fig. 7b)**
   Bestimmung des Verlusts an Aktivität durch Lagerung von Plättchen-reichem Plasma bei Raumtemperatur durch Messung der Aggregation mit ADP als Agonist mit und ohne Dauerrühren

## Patentansprüche

1. Verfahren zur Messung der Aggregation von Blutplättchen, wobei turbidimetrische, nephelometrische oder elektromagnetische Methoden zur Messung der Aggregatbildung eingesetzt werden und ein Reaktionsgemisch in einer ersten Reaktionsphase durchmischt wird und in einer auf die erste folgenden, zweiten Reaktionsphase nicht durchmischt wird, wobei die Messung bevorzugterweise in der zweiten Reaktionsphase erfolgt.

2. Verfahren nach Anspruch 1 wobei die Durchmischung durch Rühren, Schütteln, Vibrieren oder Ultraschall erfolgt.

3. Verfahren gemäß Anspruch 1 oder 2 zur Bestimmung der Aggregation von physiologisch aktiven Blutplättchen.

4. Verfahren gemäß Anspruch 1 oder 2 zur Bestimmung der Aggregation von fixierten Blutplättchen, insbesondere im Ristocetin-Kofaktor Test.

5. Verfahren gemäß einem der Ansprüche 1 bis 3, wobei als Probe Vollblut, Plättchen-reiches Plasma, verdünntes Plättchen-reiches Plasma, oder gereinigte Thrombozyten für die Messung eingesetzt werden.

6. Verfahren gemäß einem der Ansprüche 1 bis 5, wobei die Durchmischungszeit, die notwendig ist, um die Aggregationsreaktion auszulösen, bestimmt wird.

7. Verfahren nach Anspruch 6, zur Bestimmung der Sensitivität von Blutplättchen gegenüber Plättchenaktivatoren wie z. B. Ristocetin, Kollagen, ADP, Epinephrin oder Arachidonsäure.

8. Verfahren zur Bestimmung der Stabilität von Plättchenaggregaten, wobei die Aggregation in einem Verfahren gemäß einem der Ansprüche 1 bis 5 mit einer analogen Messung bei ständigem Rühren verglichen wird.

9. Verfahren gemäß einem der Ansprüche 1 bis 8, wobei vor der Durchmischungsphase eine Inkubationsphase ohne Durchmischung eingefügt ist.

10. Verfahren gemäß einem der Ansprüche 1 bis 9, wobei vor der Durchmischungsphase ein Ausgangsmeßwert bestimmt wird.

11. Verfahren gemäß einem der Ansprüche 1 bis 10, wobei die Aggregation von Blutplättchen und Partikeln gemessen wird.

12. Verfahren gemäß einem der Ansprüche 1 bis 11, wobei anstelle von Blutplättchen andere Zellen, Membranvesikel oder künstliche Partikel verwendet werden.

13. Verfahren gemäß einem der Ansprüche 1 bis 12, wobei die Durchmischung nicht komplett gestoppt wird sondern die Durchmischung auf eine geringere Intensität eingestellt wird.

14. Verfahren gemäß einem der Ansprüche 1 bis 13, wobei mehrere durchmischte und nicht-durchmischte Phasen aufeinander folgen.

## Claims

1. A method for measuring the aggregation of blood platelets, where turbidimetric, nephelometric or electromagnetic methods are employed for measuring the aggregate formation, and a reaction mixture is mixed in a first reaction phase, and is not mixed in a second reaction phase following the first, where the measurement is preferably carried out in the second reaction phase.

2. The method as claimed in claim 1, where the mixing takes place by stirring, shaking, vibrating or ultrasound.

3. The method as claimed in claim 1 or 2 for determining the aggregation of physiologically active blood platelets.

4. The method as claimed in claim 1 or 2 for determining the aggregation of fixed blood platelets, in particular in the ristocetin cofactor test.

5. The method as claimed in any of claims 1 to 3, where whole blood, platelet-rich plasma, diluted platelet-rich plasma or purified platelets are employed as sample for the measurement.

6. The method as claimed in any of claims 1 to 5, where the mixing time necessary for inducing the aggregation reaction is determined.

7. The method as claimed in claim 6 for determining the sensitivity of blood platelets to platelet activators such as, for example, ristocetin, collagen, ADP, epinephrine or arachidonic acid.

8. A method for determining the stability of platelet aggregates, where the aggregation in a method as claimed in any of claims 1 to 5 is compared with an analogous measurement with continuous stirring.

9. The method as claimed in any of claims 1 to 8, where an incubation phase without mixing is inserted before the mixing phase.

10. The method as claimed in any of claims 1 to 9, where an initial measurement is determined before the mixing phase.

11. The method as claimed in any of claims 1 to 10, where the aggregation of blood platelets and particles is measured.

12. The method as claimed in any of claims 1 to 11, where in place of blood platelets there is use of other cells, membrane vesicles or artificial particles.

13. The method as claimed in any of claims 1 to 12, where the mixing is not completely stopped but the mixing is adjusted to a lower intensity..

14. The method as claimed in any of claims 1 to 13, where there is a sequence of a plurality of mixed and unmixed phases.

## Revendications

1. Procédé de mesure de l'agrégation des plaquettes sanguines, dans lequel des méthodes turbidimétriques, néphélométriques ou électromagnétiques sont utilisées pour mesurer la formation d'agrégats, un mélange réactif étant mélangé dans une première phase de réaction et non mélangé dans une deuxième phase de réaction suivant la première, et la mesure se faisant de préférence dans la deuxième phase de réaction.

2. Procédé selon la revendication 1, le mélange se faisant par brassage, secousses, vibrations ou ultrasons.

3. Procédé selon la revendication 1 ou 2 pour la détermination de l'agrégation de plaquettes sanguines physiologiquement actives.

4. Procédé selon la revendication 1 ou 2 pour la détermination de l'agrégation de plaquettes sanguines fixées, en particulier dans le cadre du test du cofacteur de la ristocétine.

5. Procédé selon une des revendications 1 à 3, dans lequel on utilise comme échantillon pour la mesure du sang complet, du plasma riche en plaquettes, du plasma riche en plaquettes dilué ou des thrombocytes purifiés.

6. Procédé selon une des revendications 1 à 5, dans lequel on détermine la durée de mélange qui est nécessaire pour déclencher la réaction d'agrégation.

7. Procédé selon la revendication 6 pour la détermination de la sensibilité des plaquettes sanguines aux activateurs des plaquettes, par exemple la ristocétine, le collagène, l'ADP, l'épinéphrine ou l'acide arachidonique.

8. Procédé pour la détermination de la stabilité des agrégats plaquettaires, l'agrégation avec un procédé selon une des revendications 1 à 5 étant comparée à une méthode de mesure analogue sous agitation constante.

9. Procédé selon une des revendications 1 à 8, dans lequel une phase d'incubation sans mélange est ajoutée avant la phase de mélange.

10. Procédé selon une des revendications 1 à 9, dans lequel la valeur de mesure de sortie est déterminée avant la phase de mélange.

11. Procédé selon une des revendications 1 à 10, dans lequel on mesure l'agrégation des plaquettes sanguines et des particules.

12. Procédé selon une des revendications 1 à 11, dans lequel on utilise à la place des plaquettes sanguines d'autres cellules, des vésicules membranaires ou des particules synthétiques.

13. Procédé selon une des revendications 1 à 12, dans lequel le mélange n'est pas complètement arrêté, mais le mélange est réglé à une moindre intensité.

14. Procédé selon une des revendications 1 à 13, dans lequel plusieurs phases mélangées et non mélangées se succèdent.
